# EUROPEAN PATENT SPECIFICATION

(11) **EP 0 541 666 B1**
(45) Date of publication and mention of the grant of the patent: **27.12.1995**
(21) Application number: 91914262.0
(22) Date of filing: 30.07.1991
(51) Int. Cl.: A61F 2/08

(54) **IMPROVEMENTS RELATING TO SURGICAL DEVICES**
CHIRURGISCHE VORRICHTUNGEN
AMELIORATIONS APPORTEES A DES DISPOSITIFS CHIRURGICAUX

(30) Priority: 31.07.1990 GB 9016761
(43) Date of publication of application: 19.05.1993
(73) Proprietor: JOHNSON, David Paul, Failand, Bristol BS8 3UL (GB)
(72) Inventor: JOHNSON, David Paul, Failand, Bristol BS8 3UL (GB)
(74) Representative: Smith, Martin Stanley
(86) International application number: GB9101295
(87) International publication number: WO9202196

(56) References cited:
- EP-A- 0 232 049
- CH-A- 267 810
- DE-U- 8 809 612
- FR-A- 2 483 772
- US-A- 3 973 277
- US-A- 4 772 286

## Description

This invention relates to surgical devices and is concerned with an adjustable tensioning assembly for ligaments as specified in the preamble of Claim 1. Such a device is known e.g. from CH-A-267 810 or from EP-A-0 232 049.

The recent treatment of chronic knee instability due to rupture of the anterior cruciate ligament has been a mixture of ligament repair, augmentation or substitution by tendon transfer. The mediocre results and the inability of most athletes to return to their previous sporting activities in full capacity has prompted intense research into prosthetic cruciate replacement (Johnson RJ et al. 1984). Initial research centred upon the use of the patella tendon or tensor fascia larter. Subsequent research centred upon carbon fibre or dacron ligaments as scaffholds for the slow invasion by fibrous tissue (Butler DL et al 1985). Recently great interest has been shown in expanded polytetrofluroethelene or "Gore-tex" (a trademark) ligaments. This ligament was first used in a multi centre research project in 1984 and the initial good results have resulted in its wider use. However, the long term results of independent assessment suggest an incidence of chronic knee effusion and late rupture of the Gore-tex graft. As a result, the most common technique at the present time is the use of an autogenous patella tendon graft with bone harvested from the patella and the tibial tuberosity. The patella tendon graft and bone plugs are fed into tunnels within the femur and tibia and secured at each end.

One of the outstanding problems and the subject of much discussion is the isometric placement of these ligaments. Theoretically isometric placement is possible where the ligament remains at the same tension throughout the range of knee flexion. However, this is difficult to achieve in practice. Many recent projects have defined the isometric points for insertion of the drill and have demonstrated the effect of incorrect placement. However, an unsolved problem is the fixation of the ends of the ligament with sufficient strength for early motion to be allowed. Different techniques include screws, baffles, bone blocks, staples, washers, screws and more recently toggles (Amis AA 1988. Good et al 1988). These all provide support which may be unreliable and which usually require protection or cautious use for up to one year to allow adequate fibres or bony ingrowth to provide secure fixation. This delays the return to activity, particularly sports, which leads to more muscle wasting and stiffness.

The final problem, which has yet received little attention, is correct tensioning of the ligament. For the ligament to be functional it not only has to be inserted isometrically but it has to be correctly tensioned in order to allow a full range of motion. In particular, it needs to be tight enough to give stability rather than being a check rein loaded only at the extremes of motion. With some ligaments, such as the Leeds Keio ligament, maintenance of tension during insertion is technically difficult, and early reports suggest some degree of laxity is often present post-operatively. No method of insertion is believed to be currently available such that the tension may be incrementally increased while the range of motion and stability is continuously examined.

The aim of this invention is to enable a ligament to be inserted and firmly held in position in an easy and certain manner, while allowing the tension in the ligament to be adjusted to an optimum. It may even be adjustable at a later operation if necessary. It should also be usable for a prosthetic ligament graft as well as an autogenous patella tendon graft.

According to the present invention there is provided a tensioning device for ligament grafts as specified in Claim 1.

To fit such a device the bone is drilled through and the anchorage element, with one end of the ligament engaged with it, is inserted through the bore from one side. The thimble is placed to abut the opposite side of the bone, having a portion which locates it partially within the other end of the bore. The screw means are then engaged and as they are tightened the ligament is tensioned and the thimble is drawn more firmly against the bone.

Usually, there will be a pair of such devices with the other end of the ligament anchored in a similar manner, although screw-adjustability at both ends may not always be required.

The thimble is generally of hollow cylindrical form having an external flange at one end and an internal shoulder at the other end, the flange being for abutment of bone around the mouth of a drilling in which the remainder of the device is received, and the shoulder being for retention of the screw means. The flange will generally be at a slant with respect to the axis of the cylinder, preferably in the range of 45° - 60° and the screw means will have an external projection for engagement of said internal shoulder of the thimble. The screw means may have a detent in its end for turning by a tool entered through the thimble, and conveniently it takes the form of a nut engageable on a threaded portion of said anchorage element.

The anchorage element is of generally cylindrical form co-axial with the screw means to fit snugly in the bore through the bone, but having a lateral recess to accept an end of the ligament. A slot leads from said recess to said one to guide the ligament into leading substantially co-axially from said element, the portion in which the slot is formed providing an abutment for a bone fragment at the end of a harvested tendon. In another form, the recess has an upstanding abutment within the envelope of said element for the retention of the end of a prosthetic anterior cruciate graft.

For a better understanding of the invention one embodiment will now be described, by way of example, with reference to the accompanying drawing, in which:
Figure 1 is a side view, partially in longitudinal section, of a ligament tensioning device,
Figure 2 is a section on the line II-II of Figure 1,
Figure 3 shows a lead wire for use with the device,
Figure 4 is a side view of a tensioning key for use with the device, and
Figure 5 shows views of an alternative carrier that could be incorporated in the device.

Reference will be made to inner and outer ends, these being with reference to the mid-point of the assembly.

At each end the tensioning device comprises a thimble 1 outermost, an intermediate nut 2, and a carrier 3 innermost. At the left hand end the carrier 3 is shown rotated through 90° from identical orientation with the carrier at the right hand end.

Each thimble 1 is of squat cylindrical form with its outer end angled and having an external flange 4. This angle is preferably between 45° and 60° to the axis. Internally, the thimble has a thickened portion towards its inner end to form a shoulder 5.

Each nut 2 is basically a hollow internally screw-threaded cylinder with an external rim 6 at its outer end, the underside of which co-operates with the shoulder 5 of the associated thimble. At this end, diametrically opposite zones are cut away to form slots 7. At the inner end, there is an internal bevel to facilitate coupling to the carrier 3. The nuts 2 may be provided in various lengths.

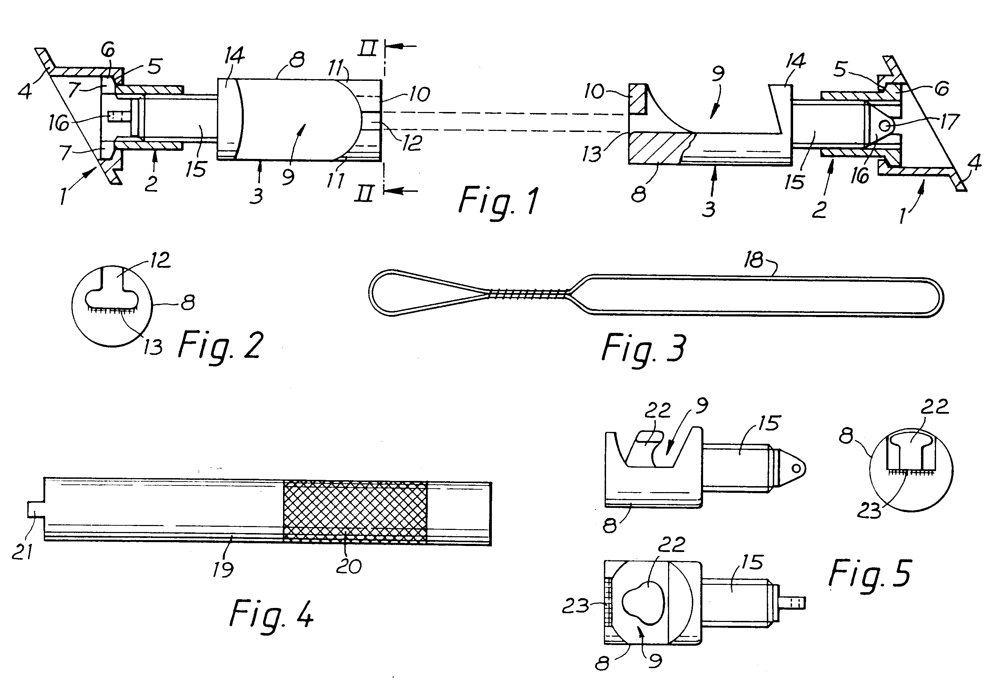

Each carrier 3 has a main body 8 having a cylindrical envelope, but this has a middle portion cut away to form a recess 9. At its inner end, the wall 10 defining the limit of the recess is shaped with re-inforcing shoulders 11 and is cut away in a T-shaped slot 12 as best seen in Figure 2. The cross arm of the T opens to the base of the recess 9 and is virtually diametral of the inner end of the carrier. Where it emerges to the end face, there is a rounded or bevelled edge 13 to reduce chafe. At the outer end of the recess 9, the end wall 14 is undercut to contain the bone fragment. Beyond that there is a co-axial stud 15 externally screw threaded to mate with the nut 2. At its coned tip, which eases entry into the nut 2, the stud 12 has a transverse lug 16 with a small hole 17.

For fitting this device the bone is drilled through with a bore corresponding to the envelope diameter of the carriers 3, which is the same as that of the non-flanged parts of the thimbles 1. A lead wire 18 is connected to one of the carriers 3 using the hole 17. A suitable configuration for the wire 15 is shown in Figure 3. The long loop is then passed through the bore, and also through the nut 2 and thimble 1 which are lightly held in place at the other end of the bore. It can also be passed through a tensioning key 19 (Figure 4) which is of hollow cylindrical form with a knurled portion 20 for a good grip, and two lugs 21 at one end to co-operate with the slots 7. The carrier 3, with a bone fragment located in the recess 9 and the attached tendon leading through the slot 12, is drawn up the bore until the stud 15 engages the nut 2, which can then be turned using the key 19 to complete the tensioning. Once that is done, the key can be removed, and then the lead wire 18.

This assumes that the ligament is already anchored at the other end. If it is not, then of course there will be no tension and the nut is simply done up a number of turns to ensure a good grip, while allowing for later adjustment.

The alternative carrier of Figure 5 differs by having a bollard 22 upstanding in the recess 9 and inclining towards the stud end. The undercut at that end is not required and both end walls have generally the same configuration with the T-slot opened out into a square one 23. This bollard enables a prosthetic anterior cruciate graft to be coupled as an alternative to an autogenous patella tendon.

## Claims

1. A tensioning device for a replacement ligament, the device comprising a bone engaging thimble (1), a generally cylindrical anchorage element (3) to which the replacement ligament is made captive, and screw means (2) located by said thimble (1) and co-operating co-axially with said element (3) at one end for drawing said element and said thimble together to tension the ligament extending from the other end, characterised in that said element (3) has a lateral recess (9) to accept and retain an end of the ligament and a wall (10) dividing said recess from said other end, a slot (12) in this wall (10) permitting the portion of the ligament adjacent the captive end to be passed laterally inwards to align substantially co-axially with said element.

2. A device as claimed in Claim 1, characterised in that said bone engaging thimble is of hollow cylindrical form having an external flange (4) at one end and an internal shoulder (5) at the other end, the flange (4) being for abutment of bone around the mouth of a drilling in which the remainder of the device is received, and the shoulder being for location and retention of the screw means (2), which has an external projection (6) for engagement of said shoulder.

3. A device as claimed in Claim 2, characterised in that the flange (4) is at a slant with respect to the axis of the thimble.

4. A device as claimed in Claim 2 or 2, characterised in that the screw means (2) has a detent (7) in its end for turning by a tool entered through the thimble.

5. A device as claimed in any preceding Claim, wherein the screw means (2) is a nut engageable on a threaded portion (15) of said anchorage element (3).

6. A device as claimed in any preceding Claim, characterised in that said wall (10) in which the slot (12) is formed provides an abutment for a bone fragment at the end of a harvested tendon.

7. A device as claimed in any one of Claims 1 to 5, characterised in that the recess (9) has an upstanding abutment (22) within the substantially cylindrical envelope of said element (3) for the retention of the end of a prosthetic anterior cruciate graft.

## Patentansprüche

1. Eine Spannvorrichtung für ein Ersatzligament, und zwar umfaßt die Vorrichtung einer knochenerfassenden Zwinge (1), ein im allgemeinen zylindrisches Verankerungselement (3), an das das Ersatzligament befestigt ist, und ein Schraubelement (2), das durch die besagte Zwinge (1) lokalisiert ist und mit dem besagten Element (3) an einem Ende koaxial zusammenwirkt, um das besagte Element und die besagte Zwinge zwecks Spannung des sich von dem anderen Ende erstreckenden Ligaments zusammenziehen, dadurch gekennzeichnet, daß das besagte Element (3) für die Aufnahme und Halterung eines Ende des Ligaments eine seitliche Aussparung (9) sowie eine Wand (10) aufweist, die die besagte Aussparung von dem besagten anderen Ende trennt, wobei ein Schlitz (12) in dieser Wand (10) es ermöglicht, den anschließend an das befestigte Ende befindlichen Teil des Ligaments seitlich einwärts zu führen, um ihn im wesentlichen koaxial mit dem besagten Element auszurichten.

2. Eine Vorrictung nach Anspruch 1, dadurch gekennzeichnet, daß die besagte knochenerfassende Zwinge von hohler zylindrischer Form mit einem Außenflansch (4) an einem Ende und einer inneren Schulter (5) am anderen Ende ist, wobei der Flansch (4) als Abstützung des Knochens ringsum die Öffnung eines Bohrlochs dient, in der der restliche Teil der Vorrichtung aufgenommen wird, und die Schulter zu Lokalisierung und Halterung des Schraubelements (2) bestimmt ist, daß zwecks Eingriff mit der besagten Schulter mit einem äußeren Vorsprung (6) versehen ist.

3. Eine Vorrichtung nach Anspruch 2, dadurch gekennzeichnet, daß der Flansch (4) im Verhältnis zu der Zwingeachse schräg liegt.

4. Eine Vorrichtung nach Anspruch 2 oder 3, dadurch gekennzeichnet, daß das Schraubelement (2) an seinem Ende einen Anschlag (7) zum Drehen eines durch die Zwinge hindurch eingeführten Werkzeugs aufweist.

5. Eine Vorrichtung nach einem der vorstehenden Ansprüche bei der das Schraubelement (2) eine Mutter ist, die mit einem Gewindeteil (15) des besagten Verankerungselements (3) in Eingriff gebracht werden kann.

6. Eine Vorrichtung nach einem der vorstehenden Ansprüche, dadurch gekennzeichnet, daß die besagte Wand (10), in der der Schlitz (12) gebildet ist, eine Abstützung für ein Knochenelement am Ende einer gewonnenen Sehne bildet.

7. Eine Vorrichtung nach einem der Ansprüche 1 bis 5, dadurch gekennzeichnet, daß die Aussparung (9) zwecks Halterng des Endes eines prosthetischen vorderen Kreuzbandtransplantats eine aufrechte Abstützung (22) innerhalb der im wesentlichen zylindrischen Hülle des besagten Elements (3) aufweist.

## Revendications

1. Dispositif de tension pour ligament de remplacement, le dispositif comprenant une virole d'engagement de l'os (1), un élément d'ancrage de forme générale cylindrique (3) auquel est fixé le ligament de remplacement, et un moyen à visser (2) positionné par ladite virole (1) et coopérant axialement avec ledit élément (3) à une extrémité pour tirer ensemble ledit élément et ladite virole de manière à tendre le ligament s'étendant de l'autre extrémité, caractérisé en ce que ledit élément (3) a un évidement latéral (9) pour recevoir et retenir une extrémité du ligament et une paroi (10) divisant ledit évidement de l'autre extrémité précitée, une fente (12) dans cette paroi (10) permettant à la partie du ligament contiguë à l'extrémité prisonnière de passer latéralement vers l'intérieur pour s'aligner substantiellement coaxialement avec ledit élément.

2. Dispositif selon la Revendication 1, caractérisé en ce que ladite virole d'engagement de l'os est de forme cylindrique creuse ayant une bride extérieure (4) à une extrémité et un épaulement intérieur (5) à l'autre extrémité, la bride (4) servant de butée à l'os autour de l'orifice d'un perçage recevant le reste du dispositif, et l'épaulement étant prévu pour le positionnement et la retenue du moyen à visser (2) qui a une saillie extérieure (6) pour l'engagement dudit épaulement.

3. Dispositif selon la Revendication 2, caractérisé en ce que la bride (4) est de biais par rapport à l'axe de la virole.

4. Dispositif selon la Revendication 2 ou 3, caractérisé en ce que le moyen à visser (2) a un moyen d'arrêt (7) dans son extrémité pour être tourné par un outil introduit à travers la virole.

5. Dispositif selon l'une quelconque des Revendications précédentes, dans lequel le moyen à visser (2) est un écrou pouvant s'engager sur une partie filetée (15) dudit élément d'ancrage (3).

6. Dispositif selon l'une quelconque des Revendications précédentes, caractérisé en ce que ladite paroi (10) dans laquelle est formée la fente (12) constitue une butée pour un fragment d'os à l'extrémité d'un tendon acquis.

7. Dispositif selon l'une quelconque des revendications 1 à 5, caractérisé en ce que l'évidement (9) a une butée en saillie (22) à l'intérieur de l'enveloppe sensiblement cylindrique dudit élément (3) pour la retenue de l'extrémité d'une greffe cruciforme antérieure prothétique.
